# EUROPEAN PATENT APPLICATION

(11) **EP 1 315 099 A1**
(43) Date of publication of application: **28.05.2003**
(21) Application number: 01943854.8
(22) Date of filing: 28.06.2001
(51) Int. Cl.: G06F 17/30, C12M 1/00, C12N 15/00

(54) **MICROBE ANALYZING SYSTEM AND METHOD, AND DATABASE**

(30) Priority: 28.06.2000 JP 2000194331
(71) Applicant: Sanyo Electric Co., Ltd., Moriguchi-shi, Osaka-fu 570-8677 (JP)
(72) Inventor: INOUE, Takakazu, Moriguchi City, Osaka 570-8677 (JP); SEKIGUCHI, Tatsuhiko, Moriguchi City, Osaka 570-8677 (JP); FUJIMURA, Kouta, Moriguchi City, Osaka 570-8677 (JP); IWAMA, Akifumi, Moriguchi City, Osaka 570-8677 (JP)
(74) Representative: Cross, Rupert Edward Blount
(86) International application number: JP0105548
(87) International publication number: WO02001412

(57) **Abstract**

A microbe analyzing system for analyzing a DNA and identifying the microbe in a sample. The user amplifies a DNA fragment of a microbe in a sample by the PCR method, creates electrophoresis data, sends the electrophoresis data from client computers (12, 14) through a communication network (10) to a server computer (16) . The server computer (16) accesses a database (16a) where electrophoresis data set of each microbe is stored, extracts the relevant microbe, and sends the result to the client computers (12, 14). The user is not required to send a sample to an analyzing company and can quickly identify the microbe on the basis of electrophoresis data.

## Description

### Technical Field

The present invention relates to a method and system for analyzing microbes such as bacteria.

### Background Art

Processors are currently being developed for processing garbage discarded from residential houses or the like to produce fertilizers. In order to produce higher quality fertilizers, information about the microbes acting within the garbage processor must be known. In addition to the garbage processing as described, there also is a need for reliably and quickly analyzing a group of microbes present in a general sample.

As a method for obtaining information about a group of microbes, for example, a group of bacteria, a method is known in which each of the bacteria in a group of bacteria is individually isolated and biochemically inspected.

However, such a method is time consuming, and, in addition, there is a problem in that bacteria that cannot be isolated cannot be analyzed.

On the other hand, as a method for amplifying the DNA of microbes, PCR (Polymerase Chain Reaction) is being employed. In PCR, by using primers and thermostable DNA polymerases having base sequences which are complementary to the base sequences at the ends of the DNA to be amplified, a cycle having three stages, a thermal transformation process, an annealing process, and an extension and reaction process is repeated to enable amplification of a DNA fragment which is substantially identical to the template DNA. PCR allows for amplification by a factor of one hundred thousand to one million of DNA of bacteria that are present only in a minute numbers.

However, PCR requires that the base sequences at the ends of one region of the template DNA be known, and therefore, DNA cannot be amplified before the types of microbes present are known.

To this end, a method known as RAPD (Random Amplified Polymorphic DNA) is being proposed in which many types of DNA fragments are simultaneously amplified from a single DNA fragment using a single primer without any information of the base sequences. In this method, by lowering the annealing temperature of the primer during the reaction of PCR and increasing the magnesium ion concentration in the reaction solution, the sequence specificity of the primer during bonding is reduced and a large volume of DNA fragments are amplified.

However, when RAPD is applied to a group of microbes including a plurality of different types of microbes, because the number of types of DNA fragments to be amplified is too large, it is difficult to specify an individual microbe included in the group of microbes.

Considering the problems of the conventional art as described above, the present inventors have proposed, in Japanese Patent Laid-Open Publication No. Hei 11-341989, an art for statistically amplifying one DNA fragment from one type of microbe, by adjusting the length of the primers. More specifically, when, for example, the length of the base of the DNA of a microbe is approximately 10⁷ bp and a group of microbes in which 10 types of microbes are present is to be measured, by adjusting the primer length so that one DNA fragment is amplified every 10⁸ bp, only one type of DNA fragment is amplified for 10 types of microbes (this method is referred to as "SSC-PCR" hereinafter).

As described, in SSC-PCR, even when a plurality of microbes are present, it is possible to amplify, on average, one DNA from one type of microbe. Therefore, SSC-PCR is very effective for garbage processing and when examining a sample in which a plurality of strains of bacteria are mixed.

In conventional microbe analysis, a user collects a sample and then sends the sample to an analysis provider (a company which provides analysis services) or the like via mail, etc., and the analysis provider extracts and amplifies DNA from the received sample through PCR or the like, and provides the analysis results to the user.

However, in such a system, because there is a possibility of alteration of the sample when the sample is sent, the sample must be strictly managed and, therefore, there had been a problem in that the process becomes complex. Moreover, in such a system, a few days to a few weeks are required from the time when the sample is sent to the analysis provider until when the analysis results are actually be obtained.

Furthermore, even when it is found that more detailed examination is necessary for a particular bacteria in the sample after reviewing the analysis results, the same sample must be sent to the analysis provider again for analysis and the analysis must be requested again. Thus, it is not possible to efficiently and quickly analyze the microbes in the sample.

### Disclosure of Invention

The present invention was conceived to solve the above described problems and one object of the present invention is to provide a system and a method for analyzing a microbe sample more efficiently than possible with the conventional art.

In order to achieve at least the object described above, according to one aspect of the present invention, there is provided an analysis system comprising a first computer and a second computer connected via a communication network, wherein the first computer transmits measurement data related to the DNA of a microbe to the second computer via the communication network; the second computer matches the measurement data transmitted from the first computer and data stored in a database and transmits the matching results to the first computer via the communication network; and the first computer outputs the matching results transmitted from the second computer. Instead of the analysis provider performing all of the steps of reception of the sample, amplification of the DNA, measurement of the amplified data (electrophoresis analysis or the like), matching of the measurement data and data in a database, and notification of the matching result (analysis result) to the user as in the conventional art, in the system of the present invention, after the user obtains measurement data, the user transmits the data from the first computer to the second computer via the communication network. The second computer accesses the database, matches the received measurement data and data in the database, and transmits the matching result to the first computer which is used by the user. Because the transmission of the measurement data, matching, and reception of the matching result can be executed in a short period of time, the user can quickly and efficiently obtain the analysis result of microbes without performing complex processes such as mailing the sample to the analysis provider. Moreover, because the measurement data, unlike a physical object such as a sample, can be easily converted to a form which can be transmitted, it is possible to easily transmit the measurement data to the second computer which may be physically farther away.

According to another aspect of the present invention, it is preferable that, in the analysis system, the measurement data related to DNA is electrophoresis data of DNA of the microbe. The user can obtain information of the microbes in the sample by creating electrophoresis data and transmitting the data to the second computer.

According to another aspect of the present invention, it is preferable that, in the analysis system, the measurement data related to DNA is numerically converted data of the electrophoresis data. Normally, the electrophoresis data indicates the presence ratio as a function of a DNA size and can unambiguously be defined by specifying the band position (DNA size) and band intensity. Because of this, by numerically converting the electrophoresis data, it is possible to reliably transmit the electrophoresis data to the second computer.

According to another aspect of the present invention, it is preferable that, in the analysis system, the measurement data related to DNA is patterned data of the electrophoresis data. The band position and band intensity can be defined as an intensity function with the variable being the DNA size. More particularly, the band position and band intensity can be defined as an intensity waveform pattern. By transmitting these patterns to the second computer, the second computer can reliably receive the electrophoresis data. The pattern can be transmitted after suitable quantization or digitization.

According to yet another aspect of the present invention, it is preferable that, in the analysis system, the electrophoresis data contains primer data. When the DNA fragments are amplified through PCR, for example, SSC-PCR, the primer which is used determines the DNA fragments to be amplified. Therefore, by transmitting electrophoresis data for each of the primers used, it is possible at the second computer to reliably search for the corresponding data. Here, when a plurality of primers (primer set) are used, it is possible to transmit the electrophoresis data corresponding to the primer set.

According to another aspect of the present invention, it is preferable that, in the analysis system, the second computer matches the electrophoresis data transmitted from the first computer and electrophoresis data for each microbe stored in the database, extracts a corresponding microbe, and transmits data of the corresponding microbe to the first computer as the matching results. By storing, in the database, electrophoresis data for each microbe, it is possible to extract a microbe corresponding to the electrophoresis data transmitted from the first computer and transmit data of the corresponding microbe (that is, data for specifying a microbe such as the name of the microbe) to the first computer, that is, to the user. The number of corresponding microbes needs not be one, and it is possible to transmit data of a plurality of corresponding microbes.

According to another aspect of the present invention, it is preferable that, in the analysis system, the first computer requests information related to the corresponding microbe to the second computer via the communication network and the second computer searches for information related to the corresponding microbe stored in the database and transmits to the first computer. When the user wishes to obtain more detailed information on the corresponding microbe, the user can quickly obtain the information by sending a request to the second computer.

According to another aspect of the present invention, there is provided a method for analyzing a microbes using a terminal and a database connected to each other through a communication network, the method comprising the steps of accessing the database from the terminal using measurement data related to the DNA of a microbe as a search key; searching for data of a microbe corresponding to the measurement data; and outputting the search result to the terminal.

According to another aspect of the present invention, it is preferable that the method further comprises the steps of accessing the database from the terminal using the type of microbe used in the measurement as a search key and searching for data of a microbe corresponding to the measurement data and the type of microbe. When the type of microbes is known in advance, it is possible to efficiently perform the search according to the type of microbes.

According to another aspect of the present invention, it is preferable that the method further comprises the steps of accessing the database from the terminal using a set of primers (primer set) used in the measurement as a search key, and searching for data of a microbe corresponding to the measurement data and the set of primers.

According to the another aspect of the present invention, it is preferable that, in the method for analyzing a microbe, the measurement data related to DNA is electrophoresis data of the microbe.

According to another aspect of the present invention, it is preferable that in the method for analyzing a microbe, the electrophoresis data is electrophoresis data of a DNA fragment amplified, on average, from one DNA fragment per one type through PCR, that is, electrophoresis data obtained through SSC-PCR. In this manner, even when a plurality of types of microbes are present in the sample, each microbe can be easily identified.

According to another aspect of the present invention, it is preferable that the method further comprises the steps of requesting detailed information from the terminal to the database based on the search result, and outputting, on or through the terminal, the detailed information transmitted from the database.

According to another aspect of the present invention, there is provided a computer readable database for storing electrophoresis data of DNA fragments of microbes amplified using a plurality of primers, the stored data sorted (differentiated) according to each microbe. By accessing the database, the user can identify a microbe from the electrophoresis data of the microbe.

According to another aspect of the present invention, it is preferable that, in the database, the electrophoresis data are sorted or differentiated for a basic primer set and for detailed information and stored. In this manner, for example, the database for the basic primer set can be used for a primary search and the detailed database can be used for a secondary search, allowing for more efficient search.

According to another aspect of the present invention, it is preferable that, in the database, the electrophoresis data are differentiated or sorted for each type of microbe and stored. In this manner, when the user knows the type of microbes in which they are interested, the user can more efficiently conduct a search compared to a case when searching through all of the microbes.

According to another aspect of the present invention, there is provided a method for analyzing a microbe, comprising the steps of receiving data related to the DNA of a microbe transmitted via a communication network; matching the received data with data stored in a database; and transmitting the matching result via the communication network.

According to another aspect of the present invention, it is preferable that, in the method for analyzing a microbe, the data related to DNA is electrophoresis data of the DNA of the microbe. According to another aspect of the present invention, it is preferable that, in the method for analyzing a microbe, electrophoresis data for each microbe is stored in the database; received electrophoresis data and data stored in the database are matched to extract a corresponding microbe; and data of the corresponding microbe is transmitted as the matching result.

In the specification, the term "computer" represents, in addition to an electronic calculator, any suitable electronic device having a data processing function, communication function, and data input/output function. Similarly, the term "communicatio network" used herein includes any suitable wired or wireless medium through which data can be transmitted.

### Brief Description of Drawings

Fig. 1 is a diagram showing a system structure in a preferred embodiment of the present invention.
Fig. 2 is a flowchart showing the overall processes in the embodiment.
Fig. 3 is an explanatory diagram of an electrophoresis image of a DNA fragment amplified using a plurality of primers.
Fig. 4 is an explanatory diagram showing numerically converted data of an electrophoresis image.
Fig. 5 is an explanatory diagram showing patterned data of an electrophoresis image.
Fig. 6 is an explanatory diagram showing a structure of a database.
Fig. 7 is an explanatory diagram showing another structure of a database.
Fig. 8 is a flowchart showing processes in another embodiment of the present invention.
Fig. 9 is an explanatory diagram showing another structure of a database.
Fig. 10 is an example screen (1) displayed on a client computer.
Fig. 11 is an example screen (2) displayed on a client computer.
Fig. 12 is an example screen (3) displayed on a client computer.
Fig. 13 is an example screen (4) displayed on a client computer.
Fig. 14 is an example screen (5) displayed on a client computer.
Fig. 15 is an example screen (6) displayed on a client computer.
Fig. 16 is an example screen (7) displayed on a client computer.
Fig. 17 is an example screen (8) displayed on a client computer.
Fig. 18 is an example screen (9) displayed on a client computer.

### Best Mode for Carrying Out the Invention

The preferred embodiment of the present invention will now be described with reference to the drawings.

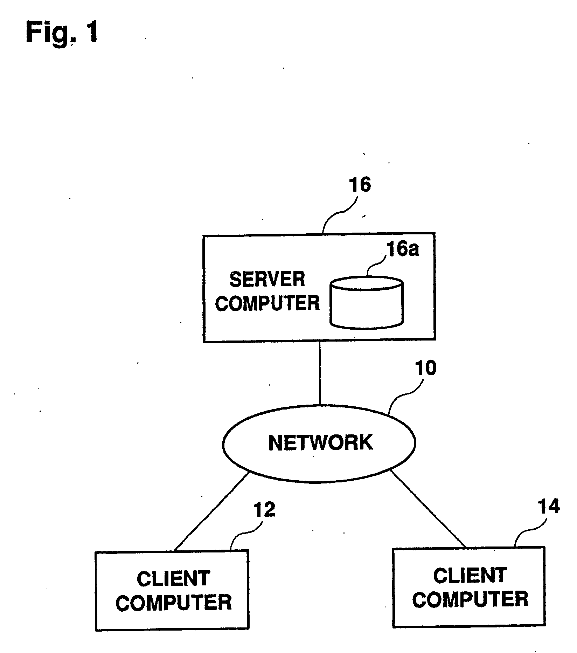

Fig. 1 is a diagram showing a system structure according to the embodiment. Client computers 12 and 14 and a server computer 16 are connected to a network 10. The client computer 12 or 14 is a terminal used by a user when requesting an analysis. The server computer 16 may be owned by an analysis provider who conducts the analysis. The client computers 12 and 14 may be general-purpose personal computers or any suitable equipment having a CPU, an input/output section, and a communication interface. The server computer 16 includes a database 16a for storing data related to the DNA of microbes, and the user can access the database 16a through the network 10. The communication network 10 can be constructed, for example, as a portion of the Internet. The server computer 16 can include a WWW server having web pages described in HTML and the user can access the database by requesting a web page of the server computer 16 using a predetermined URL. For accessing the database 16a, the user may be asked to input an ID and a password. Also, the access to the database may be charged through a predetermined method. The communication network 10 need not be formed entirely by a wired network and a portion of the communication network 10 may be wirelessly connected.

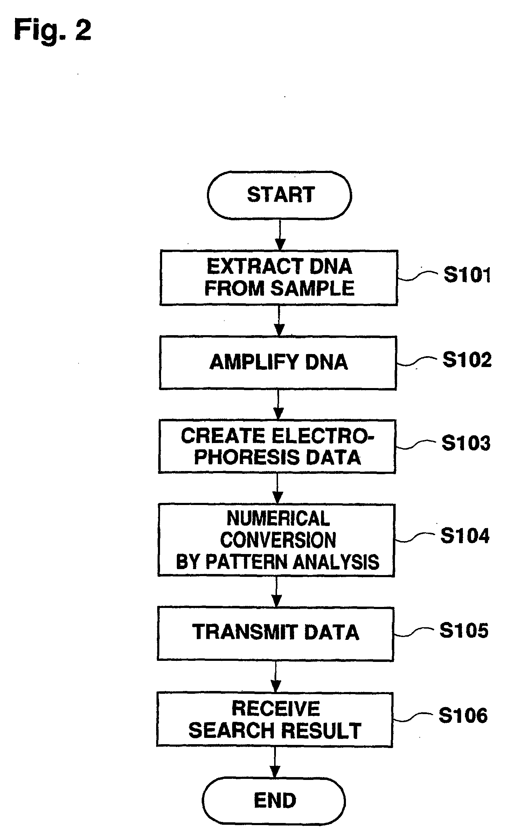

Fig. 2 shows a flowchart showing the overall process of microbe analysis according to the embodiment. First, a user extracts DNA of microbes from a sample such as, for example, garbage; human stool, skin, or saliva, etc. (step S101). Then, the DNA fragments of the microbes are amplified through SSC-PCR using a plurality of primers (primer set) so that, on average, only one type of DNA for different microbes and having differing lengths is amplified (step S102). It is preferable that the decision on which primer set is to be used for amplifying the DNA fragments be made in advance. For example, it is preferable to determine in advance a plurality of basic primers as a basic primer set and, in addition, other sets in which several primers are combined (primer set A, primer set B, . . .). In this manner, when a user amplifies DNA fragments of microbes in a sample, the user can amplify first using the basic primer set.

After the DNA fragments of microbes are amplified using a primer set, the amplified DNA fragments are subjected to the gel electrophoresis to obtain an electrophoresis image (electrophoresis data) (step S103).

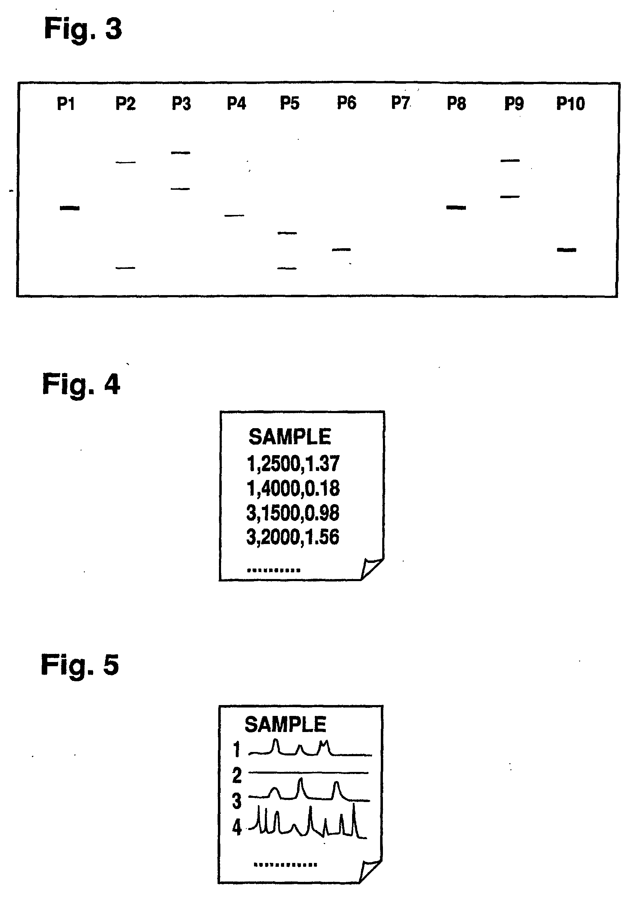

Fig. 3 shows an example of an electrophoresis image obtained in the manner described above. In Fig. 3, P1 ∼ P10 indicate the types of primers used in amplification. This set of (P1, P2, . . . P10) is, for example, determined as the basic primer set. In Fig. 3, the vertical axis represents the length of DNA fragments and the length of DNA fragment becomes shorter at the lower side of the figure. Although not shown in the figure, it is preferable that an electrophoresis image of DNA size makers be added in the direction along the vertical axis (for example, at both right and left end sections). In this manner, it is possible to easily read the size of the DNA fragment amplified by each primer. In the figure, bands of DNA fragments amplified by each primer appear. Because it is known, for each microbe, which primer amplifies the DNA fragments by what amplification, it is possible to identify, from the electrophoresis image shown in Fig. 3, to which microbe the DNA fragment belongs.

Conventionally, all of the processes from the creation of such electrophoresis image to the identification of the microbe have been performed by the analysis company, but in the embodiment the user performs processes up through the creation of the electrophoresis image, and the user then accesses, through the communication network 10, a server computer of the analysis provider which has a database related to microbe DNA, to match with their data with that stored database so that analysis can be performed substantially in real time.

For this purpose, the user must extract, from the obtained electrophoresis data, data for using the database 16a, that is, a search key for searching through the database 16a. Because this search key is transmitted through the communication network 10, it is necessary to use a search key which has minimum amount of data while representing the electrophoresis image with sufficiently high precision.

Referring back to Fig. 2, after an electrophoresis image (electrophoresis data) is obtained, the user analyzes the patterns in the electrophoresis image and converts the electrophoresis data into numerical data (step S104). This numerical conversion may be performed, for example, as follows. An electrophoresis image to which a DNA size marker is added is converted into image data using a scanner and is captured into a computer. After the image data is captured by the computer, the position and intensity of the band for each primer is read using appropriate software. The read data is then output in the form of numerical values.

Fig. 4 shows an example of numerical data obtained by analyzing an electrophoresis image. In Fig. 4, {1, 2500, 1.37) indicates that the primer number is 1 (P1), the band position is 2500, and the band intensity is 1.37. Similarly, {1, 4000, 0.18} indicates that the primer number is 1 (P1), the band position is 4000, and the band intensity is 0.18, while {3, 1500, 0.98} indicates that the primer number is 3 (P3), the band position is 1500, and the band intensity is 0.98. In this manner, by forming sets of {primer number, band position, band intensity}, the bands for all primers can be converted into numerical values. The obtained numerical data can further be binarized.

After the electrophoresis image is numerically converted through the processes as described above, the numerical data is transmitted from the computer 12 (or computer 14) to the server computer 16 (step S105). At the server computer 16, the database 16a is searched according to the received numerical data, and a microbe corresponding to the received electrophoresis data is extracted.

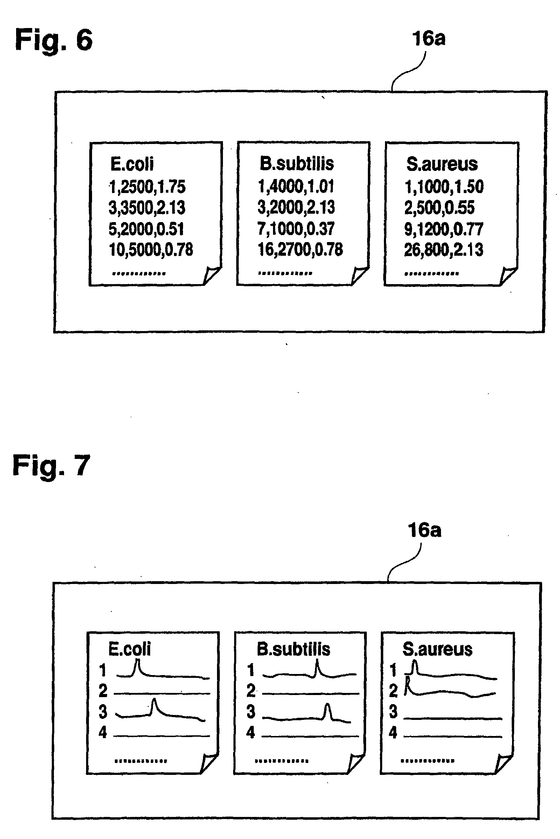

Fig. 6 schematically shows a structure of data to be stored in the database 16a. The database is divided according to individual microbe (for example, E. coli, B. subtilis, S. aureus, etc.) and stores numerical data for the electrophoresis data of each microbe. The numerical data shown in Fig. 6 is obtained by amplifying DNA fragments using the basic primer set. It is preferable that, for each of the primer sets, numerical data is stored. It is possible to extract the corresponding microbe at the server computer 16 by executing correlation calculation between the numerical data transmitted from the computer 12 (or computer 14) and the numerical data for the microbes and finding the microbe which has the highest correlation value. As a method for extraction, in addition to extracting only the one microbe having the highest correlation value, it is also possible to extract all of the microbes which have a correlation value greater than or equal to a threshold value. The server computer 16 returns the extraction results to the computer 12 (or computer 14).

The computer 12 (or computer 14) receives the data transmitted from the server computer 16 (step S106) and generates output to a display, a printer, or other output device. By checking this results, the user can quickly recognize which microbes may be present in the sample.

It is also possible that, instead of converting the electrophoresis image into numerical data at step S104, the electrophoresis image may be converted into predetermined data through other methods.

Fig. 5 shows patterned data of an electrophoresis image. The electrophoresis image is read using a scanner and the captured image is input into a computer. In the computer, using software, brightness is continuously read along a certain line (for example, the center line) for each primer. The brightness level is high at positions where a band is present and is low at positions where no band is present. The brightness level appears as a continuous waveform data and pattern data is obtained for each primer. This pattern can be quantized. The pattern data thus obtained is transmitted to the server computer 16.

The server computer 16 matches the received pattern data with the pattern data stored in the database 16a. Fig. 7 shows an example structure of data stored in the database 16a. Similar to the data shown in Fig. 6, the data in Fig. 7 is divided according to microbe and stored as pattern data for electrophoresis images obtained by amplifying DNA fragments for each microbe by predetermined primer sets. It is also preferable that the pattern data be prepared for each primer set. The server computer 16 compares the received pattern data and the pattern data for each microbe, executes a correlation calculation (for example, multiplication between the patterns), extracts only the microbe having the highest correlation or microbes having correlation greater than or equal to a threshold, and transmits microbe data to the computer 12 (or computer 14) . With this method as well, the user can quickly obtain information as to which microbes which may be present in the sample.

In the embodiment, it is also preferable that, after accessing the database 16a and receiving the results, the user further accesses the database 16a using the computer 12 (or computer 14) based on the received results, for requesting more detailed information. This is useful, for example, when the analysis results indicate a plurality of possible microbes and the user wishes to confirm which of the microbes are actually present in the sample.

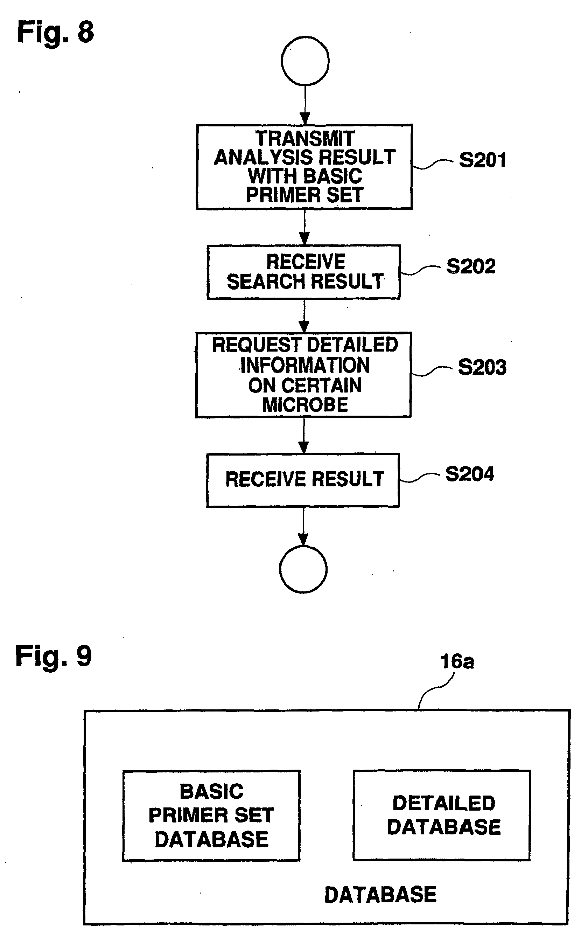

Fig. 8 shows a flowchart of the processes in this case. These processes correspond to the steps S104 ∼ S106 in Fig. 2. First, a user creates electrophoresis data using the basic primer set and transmits the data to the server computer 16 (step S201) . The server computer 16 searches through the database based on the received data and returns the corresponding microbe data to the computer 12 (or computer 14). The search result is returned, for example, in the form of a list of the corresponding microbes and correspondence probability (which is equal to the correlation). The computer 12 (or computer 14) receives the result from the server computer 16 and displays on the display or the like (step S202) . The user views the result output to the computer 12 (or computer 14) and, when the user wishes, for example, to obtain more detailed information of the microbe having the highest correspondence probability, the user requests detailed information of the microbe for which the user wishes to obtain information, to the server computer 16 (step S203). More specifically, when the search result indicates E. coli 95%, B. subtilis 70%, and S. aureus 50%, the user accesses the database 16a using E. coli as a search key. The server computer 16 searches the database 16a for detailed data for the requested microbe, and returns the results to the computer 12 (or computer 14) . By checking the detailed data (step S204), the user can quickly obtain information regarding, for example, a primer set that would identify E. coli and data for electrophoresis image which would appear when the amplification is performed using that primer set. Thus, the user can efficiently proceed with additional analysis or the like.

Fig. 9 shows another data structure in the database 16a. In addition to the database for the basic primer set, a detailed database for each microbe is stored in such a manner that will facilitate response to a user request. The electrophoresis data for primer sets other than the basic primer set and data of the primer sets which are most effective for each microbe can be stored in such a detailed database.

As described, in the embodiment, the user analyzes the electrophoresis data of microbes and accesses the database 16a through the communication network 10 after obtaining the analysis result to identify the corresponding microbe. Thus, it is possible to quickly obtain information of microbes that may correspond without strictly managing the sample for an extended period of time in order to send the sample to an analysis provider as required in the conventional art.

Moreover, according to the embodiment, because it is possible to obtain necessary detailed information approximately in real time by searching through the database 16a, it is possible to reduce the time period required for ultimately determining which microbes are present in the sample.

For efficient search, it is necessary that the database 16a be updated frequently to store the most recent data, and it is also preferable that the database be classified so that a search can be performed according to the type of the sample to be inspected. For example, the database can be divided for each of the types of microbes such as, for example, laxative bacteria, pathogenic bacteria, enterobacteria, etc., in addition to the division by microbes and by primer sets.

The operation in the embodiment will now be described using example screens displayed on the display of a computer 12 (or computer 14).

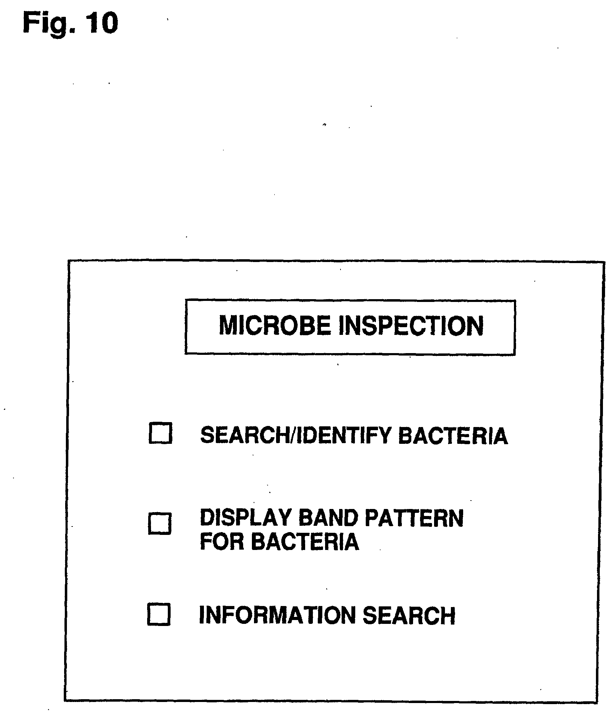

Fig. 10 shows an example initial screen to be displayed on a computer 12. This screen is displayed, for example, using a WWW browser. On the web page may be displayed fields such as "search/identify bacteria", "display band patterns for bacteria", "information search", etc. When a user has obtained electrophoresis data using a certain primer set (normally, the basic primer set is used for the initial search), the user selects the "search/identify bacteria" item.

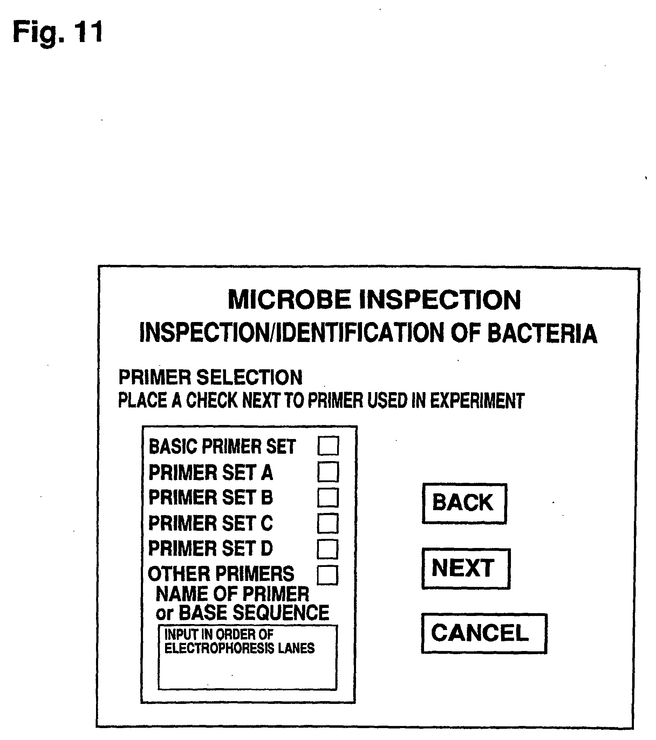

Fig. 11 shows an example display of a web page transmitted from the server computer 16 when the user selects the "search/identify bacteria" item. This screen is created for the user to select the primer set which has been used, and displays, as a list, a plurality of primer sets in addition to the "basic primer set". The user selects and transmits the primer set which was used for amplifying the DNA fragments. .

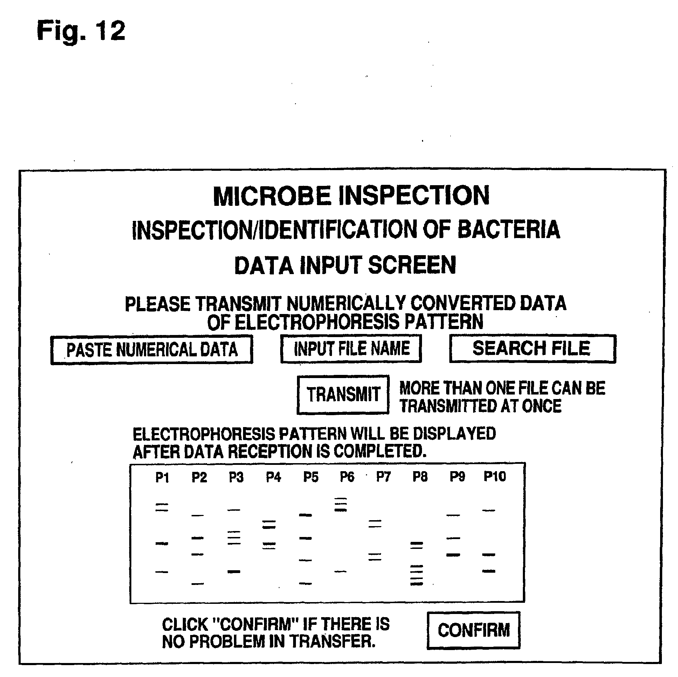

After the user transmits the primer set, the server computer 16 transmits a web page which asks for the user to input the electrophoresis data. Fig. 12 is an example of such input screen. With a message of "please transmit numerically converted data of electrophoresis pattern", a description of a plurality of methods are displayed for transmitting the numerically converted data. In Fig. 12, in addition to a method of transmitting the obtained numerically converted data by copying and pasting, a method is shown for transmitting the numerically converted data by attaching a file to which the numerically converted data are stored. After the numerically converted data is transmitted, the server computer 16 reconstructs the electrophoresis image from the received numerical data and returns to the computer 12. The user can reconfirm the transmitted numerical data by viewing the reconstructed electrophoresis image.

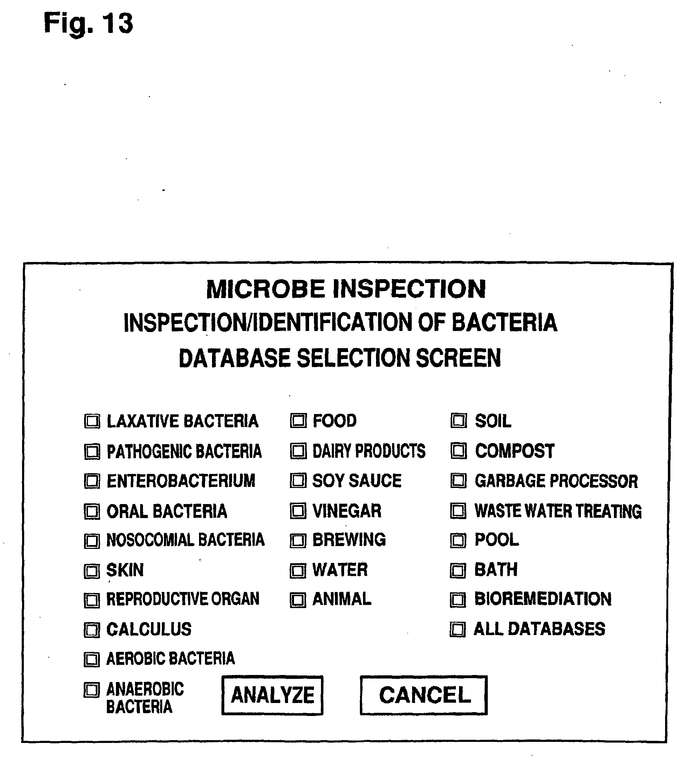

Fig. 13 shows an example screen transmitted from the server computer 16 after the numerically converted data is transmitted. This screen asks the user for the type of database to be used in the search. In the figure, the database is divided according to each type of microbes, for example, "laxative bacteria", "pathogenic bacteria", "enterobacteria", "oral bacteria", "food", "waste water processing", etc. For example, for a stool sample obtained from a patient having diarrhea symptoms, the user can select "laxative bacteria". Similarly, when a sample was obtained from a garbage processor, the user can select "garbage processor". After the type of microbe or sample is selected, the user can transmit by pressing the "analysis" button to initiate a search through the database 16a using the designated primer set and the type of microbes.

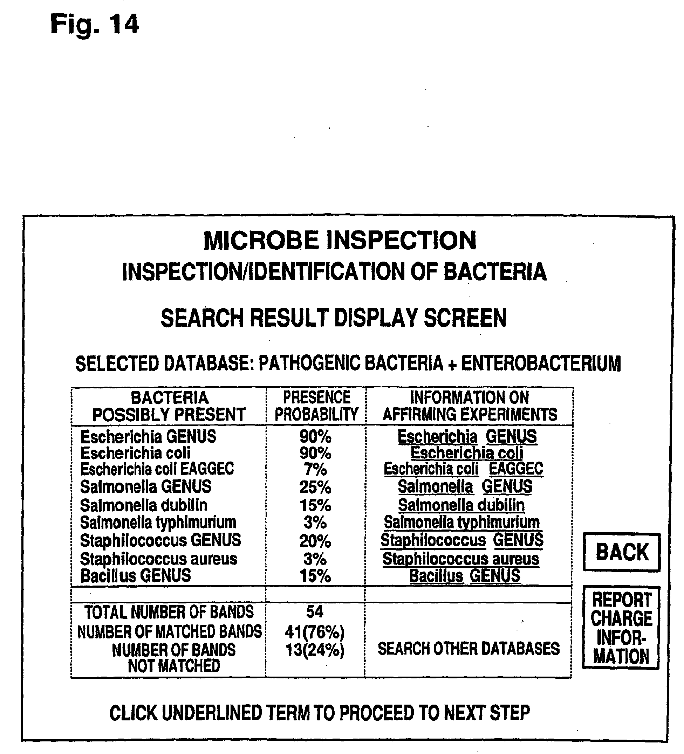

Fig. 14 shows an example screen displaying the search result. In this example screen, a case is considered in which the selected primer set is the basic primer set and the selected types of the microbes are pathogenic bacteria and enterobacteria. The names of microbes (bacteria) that may be present within (or may correspond to) the sample are displayed with the probability of presence in a form of a list. A button (or a tag) for requesting information on experiments for confirming the bacteria type is provided for each of the microbes that may be present, and a user who wishes more detailed information about the microbes can request detailed information from the server computer 16 by manipulating these buttons. Moreover, in addition to the list of microbes that may be present, the number of bands that did not match and respective percentages are also shown. When these numbers are large, the user can search through other databases.

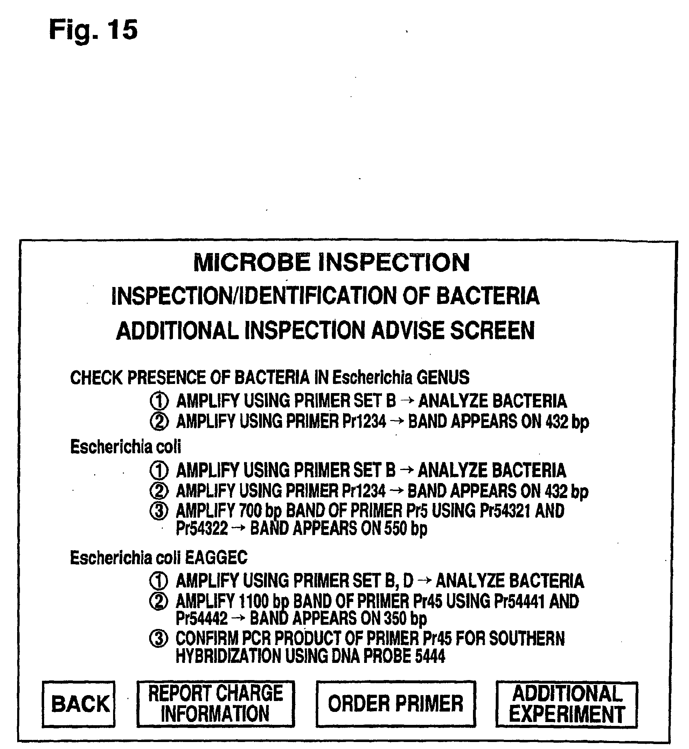

Fig. 15 shows an example screen transmitted from the server computer 16 when the user requests detailed information on microbes (bacteria) for which the probability of presence is high or microbes that are otherwise of interest to the user. For each microbe, primer sets which can be used to more affirmatively confirm the presence of that microbe, and band positions that would be expected to appear after the amplification are displayed so that the user has a useful guideline for additional experiments. In addition, in consideration of cases wherein the user does not have the necessary primer set in hand, it is preferable to display , as shown in the figure, a button for ordering the primer set from the analysis provider or other source.

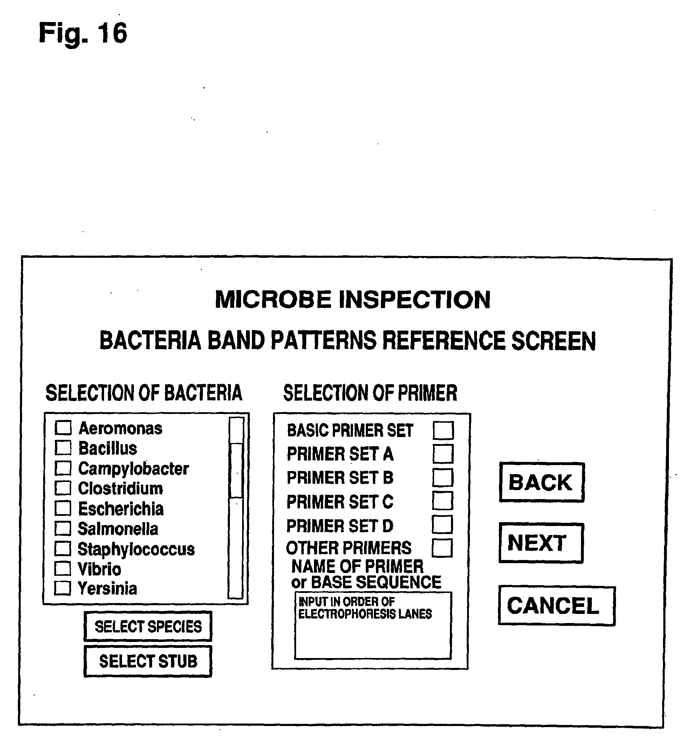

Fig. 16 shows an example web page which is transmitted from the server computer 16 when the user selects "display band patterns for bacteria" in the initial screen shown in Fig. 10. A screen for inputting the name of the desired microbe (name of bacteria) and the primer set which has been used is displayed. The microbe can be selected by species and strains.

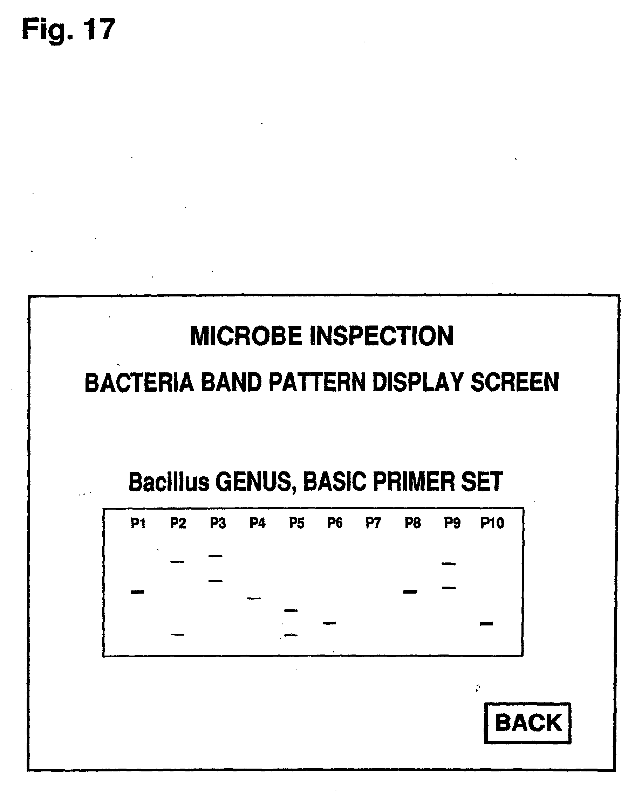

Fig. 17 shows an example screen transmitted from the server computer 16 when the user selects Bacillus genus and the basic primer set on the screen of Fig. 16. As described above, the database 16a stores the electrophoresis data (numerically converted data, pattern data, or image data of the image) for each microbe and each primer set. The server computer 16 displays electrophoresis images of microbes that are found through a search using the conditions designated by the user as the search key. The user can use these electrophoresis images as a reference for specifying the microbes.

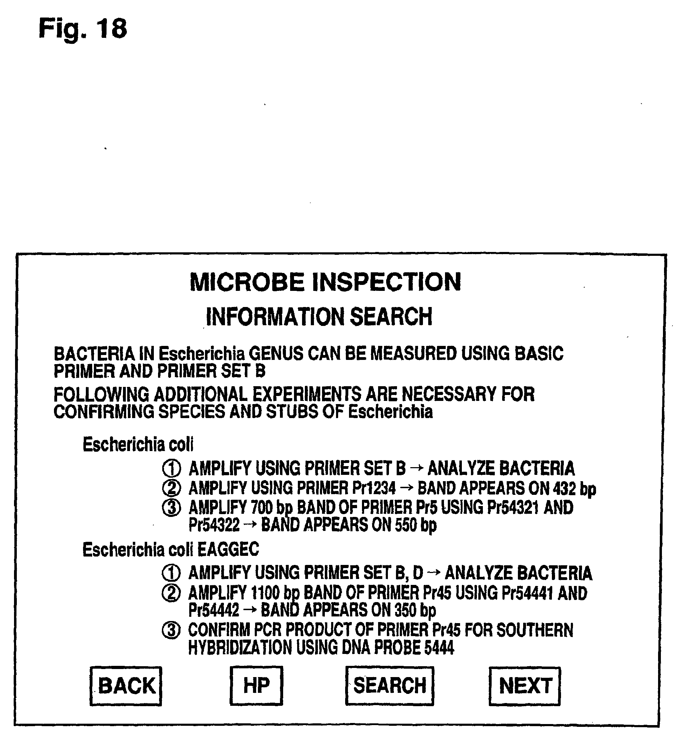

Fig. 18 shows an example web page transmitted from the server computer 16 when the user selects "information search" on the initial screen of Fig. 10. When the user wishes additional data about a certain microbe (bacteria) in addition to the identification experiment information on Fig. 14, the user can obtain detailed data from this screen.

Although a preferred embodiment of the present invention has been described, the present invention is not limited to the preferred embodiment in any way and various changes may be made. For example, the server computer 16 may be configured to enable charging, each user based on the number of databases employed in each search or the number of accesses, and the charge information can be added to the search result screen. It is also possible to provide a system in which the database related to the basic primer set is made public and the database storing data of other primer sets or detail information database is only accessible by affiliated members. It is also possible that the electrophoresis data transmitted from the user to the server computer 16 or the search results transmitted from the server computer 16 to the user computer be suitably encrypted and transmitted. Moreover, the number of database needs not be one, and the server computer 16 may access the databases of other analyzing companies when there is no data on the corresponding microbes in the database managed by the server computer 16, and transmit the result to the user.

As described, according to the present invention, the user can quickly identify a group of microbes present in a sample.

In addition, because the analysis of microbes is executed in approximately real time, feedback of the analysis results for application to the next analysis can be facilitated, allowing for an efficient analysis.

## Claims

1. A system for analyzing a microbe comprising:
a first computer and a second computer connected via a communication network; wherein
said first computer transmits measurement data related to the DNA of a microbe to said second computer via said communication network;
said second computer matches saidmeasurement data transmitted from said first computer and data stored in a database and transmits the matching results to said first computer via said communication network; and
said first computer outputs said matching results transmitted from said second computer.

2. A system for analyzing a microbe according to claim 1, wherein
said measurement data is electrophoresis data of the DNA of said microbe.

3. A system for analyzing a microbe according to claim 2, wherein
said measurement data is numerically converted data of said electrophoresis data.

4. A system for analyzing a microbe according to claim 2, wherein
said measurement data is patterned data of said electrophoresis data.

5. A system for analyzing a microbe according to claim 3, wherein
said electrophoresis data contains primer data.

6. A system for analyzing a microbe according to claim 2, wherein
said second computer matches said electrophoresis data transmitted from said first computer and electrophoresis data for each microbe stored in said database, extracts a corresponding microbe, and transmits data of said corresponding microbe to said first computer as said matching results.

7. A system for analyzing a microbe according to claim 6, wherein
said first computer sends a request for information related to said corresponding microbe to said second computer via said communication network; and
said second computer searches for information related to said corresponding microbe stored in said database and transmits a reply to said first computer.

8. A method for analyzing a microbe using a terminal and a database connected to each other through a communication network, said method comprising the steps of:
accessing said database from said terminal using measurement data related to the DNA of a microbe as a search key;
searching for data of a microbe corresponding to said measurement data; and
outputting the search result to said terminal.

9. A method for analyzing a microbe according to claim 8, further comprising the steps of:
accessing said database from said terminal using the type of microbe used in the measurement as a search key; and
searching for data of a microbe corresponding to said measurement data and said type of microbe.

10. A method for analyzing a microbe according to claim 8, further comprising the steps of:
accessing said database from said terminal using a set of primers used in the measurement as a search key; and
searching for data of a microbe corresponding to said measurement data and said set of primers.

11. A method for analyzing a microbe according to claim 8, wherein said measurement data is electrophoresis data of said microbe.

12. A method for analyzing a microbe according to claim 11, wherein
said electrophoresis data is electrophoresis data of a DNA fragment amplified, on average, from one DNA fragment per one type through PCR.

13. A method for analyzing a microbe according to claim 8, further comprising the steps of:
transmitting a request for detailed information from said terminal to said database based on said search result; and
outputting, from said terminal, said detailed information transmitted from said database.

14. A computer readable database for storing electrophoresis data of DNA fragments of microbes amplified using a plurality of primers, said stored data being sorted according to microbe.

15. A database according to claim 14, wherein
said electrophoresis data is sorted according to basic primer set and according to detailed information and stored.

16. A database according to claim 14, wherein
said electrophoresis data is sorted according to species of microbe and stored.

17. A method for analyzing a microbe, comprising the steps of:
receiving data related to the DNA of a microbe, said data transmitted via a communication network;
matching the received data with data stored in a database; and
transmitting the matching result via said communication network.

18. A method for analyzing a microbe according to claim 17, wherein
said data related to the DNA of a microbe is electrophoresis data of the DNA of said microbe.

19. A method for analyzing a microbe according to claim 18, wherein
electrophoresis data for each microbe is stored in said database;
received electrophoresis data and data stored in said database are matched to extract a corresponding microbe; and
data of the corresponding microbe is transmitted as said matching result.
